# EUROPEAN PATENT APPLICATION

(11) **EP 2 463 838 A1**
(43) Date of publication of application: **13.06.2012**
(21) Application number: 11192256.3
(22) Date of filing: 06.12.2011
(51) Int. Cl.: G08B 21/12, G08B 27/00

(54) **System and method of providing compliance and alerting of toxic gas exposure for health monitoring and plant maintenance**

(30) Priority: 10.12.2010 US 965309
(71) Applicant: Honeywell International, Inc., Morristown, NJ 07962-2245 (US)
(72) Inventor: Kumar, Nukala Sateesh, Morristown, NJ 07962-2245 (US); K, Arunkumar, Morristown, NJ 07962-2245 (US); Stinson, Sean, Morristown, NJ 07962-2245 (US); Dasari, Kondaiah, Morristown, NJ 07962-2245 (US)
(74) Representative: Buckley, Guy Julian

(57) **Abstract**

An apparatus to obtain zoned real-time gas concentration and location information from a plurality of gas detectors and provide compliance information along with exposure trends and warning information. Acquired concentration and location information can be stored in a common memory unit for efficient trend analysis and generation of compliance information.

## Description

### FIELD

The invention pertains to systems that monitor a region, or regions for toxic gas exposure. More particularly, the invention pertains to such systems which determine trends and identifying high risk areas, as well as implementing automated methods to provide compliance reports for regulatory agencies.

### BACKGROUND

In known gas detection systems and methods, operators in a region being monitored carry individual gas detectors. Fixedly mounted detectors might also be scattered throughout the region. In known systems manual data analysis and processes are used in generating alerts based on exposure of the operators to toxic gases in the region. Not only are such manual analysis and processes error prone, they can also produce unintentional non-compliance with regulatory standards.

A variety of regulatory standards have been established by the Occupational Safety and Health Administration (OSHA) in conjunction with the National Institute for Occupational Safety and Health (NIOSH) to provide for the health and safety of operators, or workers, exposed to toxic gases at their work. Preferably, operations in a region will be in compliance with such standards.

There is a need to continue monitoring regions where toxic gases are or might be present to protect the health and safety of the operators as well as the general safety of the associated installation or plant. Real-time collection of exposure and location information per operator would be useful in this regard along with being able to detect trends associated with zones or locations in the region being monitored.

### BRIEF DESCRIPTION OF THE DRAWINGS

Fig. 1 is an over-all diagram of a system which embodies the present invention; and

Fig. 2 is a block diagram illustrating additional details of the system of Fig. 1.

### DETAILED DESCRIPTION

While embodiments of this invention can take many different forms, specific embodiments thereof are shown in the drawings and will be described herein in detail with the understanding that the present disclosure is to be considered as an exemplification of the principles of the invention, as well as the best mode of practicing same, and is not intended to limit the invention to the specific embodiment illustrated.

In one aspect of the invention, continuous/real time data collection of gas concentrations, gas exposure data and location of operators, zone/area of operators, etc. provide a basis for analysis and prediction of potential problem, or high risk, areas of a region. In another aspect of the invention, wireless portable gas detectors can be combined with location identifying wireless access points, and common, or central storage of data collected from portable and fixed detectors. Zones from where the data or information originated can be identified for further analysis.

Advantageously, trends, or anomalies in behavior can be automatically detected and drawn to the attention of operators so that high risk areas or individuals can be recognized and addressed. Compliance in connection with operator/worker safety with OSHA or NIOSH standards can be increased.

Automatic notification and daily reports can be provided to the workers/operators and also to the plant safety team. The plant safety team can automatically be alerted to safety/near miss trends. Due diligence of plant safety team can be facilitated by flagging the risks and enabling the team to act on them to reduce the near miss rates.

Systems and methods in accordance with the invention can automatically contribute to regulatory compliance as well as provide predictive alerts as to toxic gas exposure for monitoring operator health as well as plant maintenance. For example, all the gas related data from the portable/fixed gas detectors can be collected. The gases and the exposures of the individual workers/operators to those gases can be analyzed. This enables the individuals to monitor their gas exposure and the associated limits.

Better fleet management can be achieved through data analysis. The gas data can be analyzed to identity high risk locations/areas through daily/monthly/quarterly audits. Accident prone zones can be identified with historical data and fixed detector requirements suggested. The local office of OSHA can be automatically notified (e-mailing etc.) to report accidents within 8 hrs to comply with regulatory standards.

In accordance with the invention, gas exposure data can be maintained and automatic notices sent to the worker/operator and the plant safety team. This approach is predictive in that it would avoid near miss trends. Additionally since the system can maintain the gas exposure records, their presentation and format can be customized to capture interview/survey, health data as mandated by NIOSH, and automatically presented.

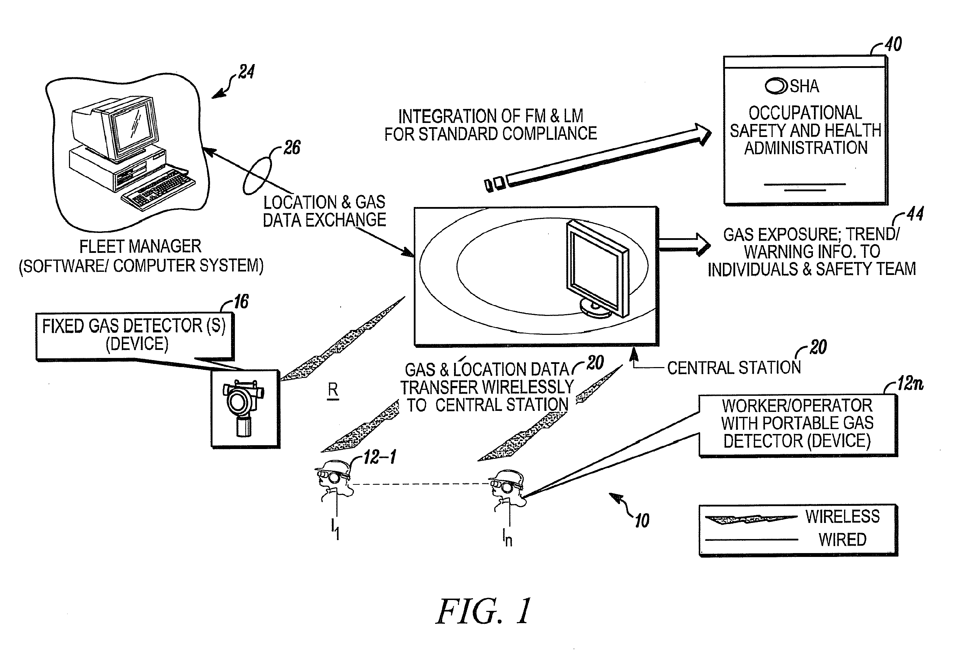

Fig. 1 illustrates a system 10 in accordance with the invention. Individuals, operators/workers I1...IN in a region R who might be exposed to one or more toxic gases, can be equipped with portable gas detectors, 12-1...12-n. Further one or more fixed gas detectors 16 can be installed in various parts of the region based on the environment and historical gas related information.

Gas concentration and location data can be transferred wirelessly to central station 20. Signals from the fixed detectors 16 can also be transferred to the central station 20.

Central station 20 can be in wired or wireless communication with a fleet manager module 24 implemented with pre-loaded executable software on a computer system. The communications are via link 26.

Fleet manager module 24 can correspond to a commercially available system marked under the "Fleet Manager" brand name by BW Technologies business unit of the assignee hereof. Such systems can store and analyze data from a plurality of gas detectors. Calibration records for the members of the plurality can be stored and updated periodically or as needed. Such records can be analyzed and trends detected relative to the on-going performance of one or more detectors as part of a gas detector management system.

Concentration and location information can be provided by central station 20 which includes a database of such information. Outputs from central station 20 and module 24 can include automatically generated OSHA compliance information 30 as well as gas exposure; trend/warning information for individuals and the safety team 34.

One such central station 20 has been disclosed and claimed in US Patent Application S.N. 12/621,713 filed November 19, 2009 and assigned to the assignee hereof.

That application, entitled, "An Alert System With Zoning Using Wireless Portable Detectors and a Central Station" is incorporated herein by reference.

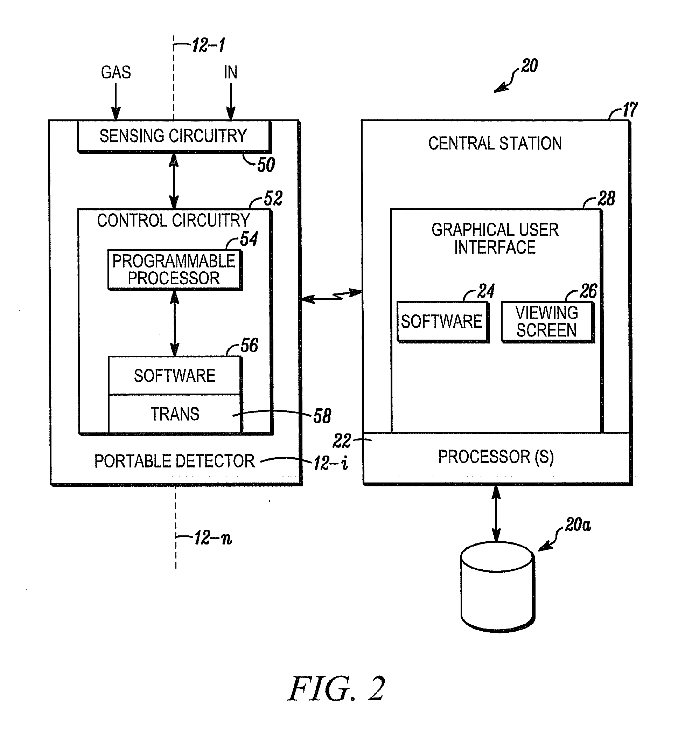

With respect to Fig. 2, system 10 includes portable detector(s) 12-1...12-n which are in wireless communication with the central station 20. As illustrated by the representative detector 12-i, the one or more portable detectors, such as 12-1...12-n can include gas sensing circuitry 50 which is coupled to control circuitry 52. The control circuitry 52 can include a programmable processor 54 and software 56, stored on a local computer readable medium, as would be understood by those of skill in the art. Ambient gases received at the portable detector 12-i can enter the sensing circuitry 50. An electrical signal, indicative of sensed gas can be coupled to the control circuitry 10.

The central station 20 can include one or more processors 22 which can be in wireless communication with the portable detector 12-1...12-n as well as the fixed detectors 16. Central station 20 can include executable configuration/monitoring software 24stored on a computer readable medium for execution by the processor (s) 22, and can include a graphical user interface 28.

The user interface 28 can include a viewing screen 26, as would be known by those of skill in the art, for displaying interactive and viewing windows. In embodiments of the present invention, the user interface 28 can be a multidimensional graphical user interface.

A storage unit 20a, coupled to processor (s) 22 can receive and store the concentration and location information from the detectors 12-1...12-n. Compliance and regulatory information can also be stored in a data base in storage unit 20a. The central station 20 can then transfer that information to module 24 for analysis and forwarding as automatically generated compliance information 40, and exposure and trend information 44.

In methods in accordance with the present invention, the central station 20 can configure a predefined Region R into multiple zones. During configuration of the predefined region R, a zone criticality (e.g., critical, non-critical, safe assembly or high, low, medium) can be assigned to each of the zones in the region. The central station can graphically differentiate the multiple zones based on the assigned criticality of each zone.

Each wireless device 12-I in the region R can periodically send ambient condition data, such as gas data, and location data to the central station 20. The central station 20 can use data received from the wireless, such as 12-I, devices to compute the alarm level for each zone. Based on the received data, the central station 20 can determine whether a particular zone is an active zone and graphically represent that zone accordingly. Because location data is periodically sent to the central station, the central station can periodically or dynamically compute the alarm level for each zone. All such information, as discussed below, can also be forwarded to module 24 to be incorporated into on-going trend analysis.

Active zones can be zones in which an alarm condition has been detected and are deemed dangerous. For example, an active zone can be a zone in which smoke has been detected, an elevated level of heat has been detected, or a gas leak has been detected.

Based on the computed alarm level of each zone, a zoning algorithm executed by the central station 20 can determine the type of alert to be sent to a wireless device, such as device 12-i, depending on where that device is located. The central station 20 can then send alert notifications to the plurality of wireless devices 12-1...12-n in the region R or zone thereof. For example, in embodiments of the present invention, the central station 20 can send a pre-alert notification to a wireless device that is near an active zone. The central station 20 can also determine if an individual is associated with a particular wireless device 12-i is headed in the direction of an active zone and send a pre-alert notification to that person to avoid entering the active zone.

The central station can also send, for example, information alerts, emergency alerts, or warning alerts to module 24 and also the wireless devices based on the location of the zone with respect to an active zone. For example, an emergency alert can be sent to a wireless device in an active zone, and an information alert can be sent to a wireless device located within a predetermined distance from an active zone. In some embodiments of the present invention, the central station 20 can send an alert notification, such as information 44, to an emergency response team if, for example, the central station 20 does not receive confirmation that an emergency notification was received by a wireless device, such as 12-i in an active zone.

In summary, system 10 provides access and control of centralized data stored in unit 20a and available to module 24 to carry out analysis and establish trends or other warning information. Compliance information 40 can be generated by units 20, 24 based on compliance information stored in unit 20a along with acquired real-time gas concentration information and location information from detectors 12-i.

Historical information, stored in unit 20a, or module 24 can be used to improve plant maintenance. The zoning capability of central station 20 makes it possible to zone the region R of the site/plant and to set criticality, size and toxic gas concentrations for the zones for use in evaluating the real-time concentration and location information from the detectors 12-i. Systematic data 44 can be generated for taking preventive actions.

From the foregoing, it will be observed that numerous variations and modifications may be effected without departing from the spirit and scope of the invention. It is to be understood that no limitation with respect to the specific apparatus illustrated herein is intended or should be inferred. It is, of course, intended to cover by the appended claims all such modifications as fall within the scope of the claims.

## Claims

1. A system comprising:
a plurality of wireless gas detectors;
at least one processor to receive location and exposure data from members of the plurality; and
a storage device coupled to the processor to store the location and exposure data and including circuitry to analyze location and exposure data for selected trends.

2. A system as in claim 1 where the storage device includes government agency compliance information.

3. A system as in claim 2 including additional circuits to automatically provide compliance reports based on stored compliance information and the stored location and exposure data.

4. A system as in claim 3 where the processor executes at least one pre-stored control program to provide the compliance reports.

5. A system as in claim 1 where the processor includes circuitry to automatically generate alert indicators in response to the stored location and exposure data.

6. A system as in claim 5 where the circuitry tracks the location and exposure data per worker and generate the alert indicators based on pre-stored regulatory standards.

7. A system as in claim 1 where the processor establishes a plurality of zones of a region being monitored responsive to region size and toxic gas information.

8. A system as in claim 7 where the processor, in response to evaluated trends, automatically provides information to operators as to preventive actions which can be taken.

9. A system as in claim 8 where the processor executes a control program to automatically provide agency compliance information relative to the region being monitored.

10. A system as in claim 9 to associate pre-established regions with real-time gas concentrations, gas exposure, and location information to determine areas of risk.

11. A central station comprising:
circuitry that configures a plurality of zones in a predefined area;
circuitry that receives ambient condition data and location data from a plurality of wireless devices located in the predefined area;
circuitry that stores condition data and location data; and
circuitry that evaluates the stored condition data and location information for trends indicative of dangerous conditions.

12. The central station of claim 11 including a server or a personal computer.

13. The central station of claim 12 including a storage unit for concentration information and location information.

14. The central station of claim 13 further comprising circuitry that automatically generates compliance information.
